# EUROPEAN PATENT SPECIFICATION

(11) **EP 2 671 585 B1**
(45) Date of publication and mention of the grant of the patent: **13.07.2016**
(21) Application number: 12742214.5
(22) Date of filing: 30.01.2012
(51) Int. Cl.: A61K 31/485, A61P 3/00, A61P 35/00

(54) **(-)-17-(cyclopropylmethyl)-3,14ß-dihydroxy-4,5a-epoxy-6ß-[N-methyl-trans-3-(3-furyl)acrylamido]morphinan for use in treating or preventing cancer cachexia**
(-)-17-(Cyclopropylmethyl)-3,14ß-Dihydroxy-4,5a-Epoxy-6ß-[N-Methyl-trans-3-(3-Furyl)acrylamido]Morphinan zur Verwendung bei der Behandlung oder Vorbeugung von Krebskachexie
(-)-17-(cyclopropylmethyl)-3,14ß-dihydroxy-4,5a-epoxy-6ß-[N-methyl-trans-3-(3-furyl)acrylamido]morphinane utilisé dans le traitement la prevention de la cachexie due au cancer

(30) Priority: 31.01.2011 JP 2011018021
(43) Date of publication of application: 11.12.2013
(73) Proprietor: Toray Industries, Inc., Tokyo, 103-8666 (JP)
(72) Inventor: SUZUKI, Tomohiko, Kamakura-shi, Kanagawa 248-8555 (JP); YOSHIZAWA, Yoshitake, Kamakura-shi, Kanagawa 248-8555 (JP); HIRAKATA, Mikito, Kamakura-shi, Kanagawa 248-8555 (JP)
(74) Representative: Kador & Partner
(86) International application number: PCT/JP2012/051937
(87) International publication number: WO 2012/105475

(56) References cited:
- WO-A1-02/089845
- WO-A1-2011/009015
- WO-A2-2011/009020
- US-A1- 2002 165 247
- US-B2- 7 652 025
- GIOVANNI MANTOVANI ET AL: "Cancer cachexia: medical management", SUPPORTIVE CARE IN CANCER, SPRINGER-VERLAG, DE, vol. 18, no. 1, 18 August 2009 (2009-08-18), pages 1-9, XP019761355, ISSN: 1433-7339
- H. KUMAGAI ET AL: "Effect of a novel kappa-receptor agonist, nalfurafine hydrochloride, on severe itch in 337 haemodialysis patients: a Phase III, randomized, double-blind, placebo-controlled study", NEPHROLOGY DIALYSIS TRANSPLANTATION, vol. 25, no. 4, 19 November 2009 (2009-11-19), pages 1251-1257, XP055182478, ISSN: 0931-0509, DOI: 10.1093/ndt/gfp588
- MASANORI FUKUSHIMA MERCK MANUAL 18TH EDITION 2007, page 1234, XP008168959

## Description

### Technical Field

The present invention relates to a compound for use in the treatment or prevention of cancer cachexia comprising as an effective ingredient a compound having a morphinan skeleton or a pharmacologically acceptable acid addition salt thereof, wherein the compound is (-)-17-(cyclopropylmethyl)-3,14β-dihydroxy-4,5α-epoxy-6β-[N-methyl-trans-3-(3-furyl) acrylamido]morphinan.

### Background Art

Cachexia, also referred to as cachexy, is a systemic syndrome having predominant symptoms such as marked weight loss, anemia, edema, anorexia, general prostration, malaise and the like in chronic diseases such as malignant tumor, tuberculosis, diabetes; blood diseases, endocrine disorders, infections, acquired immunodeficiency syndrome and the like (Non-patent documents 1 and 2).

Among those kinds of cachexia, a particular kind of cachexia caused by malignant tumor, that is, cancer cachexia is frequently found, and said to account for about 20% of the deaths from malignant tumor (Non-patent document 3). In cancer cachexia, the progression of the cachexia significantly weakens the physical strength of patients, and thus it does not allow the patients to be treated with antitumor agents, which are generally highly toxic, and it seriously undermines the treatment of malignant tumor. Furthermore, nutritional support to the patients for ameliorating the symptoms of cachexia can conversely lead to exacerbation of malignant tumor, reduction in quality of life (QOL) and descreased lifetime. Moreover, in cases of cancer cachexia, administration of an antitumor agent may induce antitumor effects but the administration, in most cases, rather causes adverse effects of the antitumor agents such as bone marrow toxicity and the like, and consequently cachexia is not ameliorated (Non-patent document 4).

1,2-diphenylpyrrole derivatives (Patent document 1), carboxylic acid amide derivatives (Patent document 2), parathyroid hormone-related peptide antibodies (Patent document 3), ghrelin-like small molecule compounds (Patent document 4), androgen receptor modulators (Patent document 5) and the like are identified as a substance having an ameliorating effect on cachexia so far. Even at present, however, the pathogenesis of cachexia is unclear, and no therapeutic agent is effective enough to be clinically used as a therapeutic agent for cachexia.

Meanwhile, a compound having a morphinan skeleton or a pharmacologically acceptable acid addition salt thereof, which is an effective ingredient of the present invention, has been disclosed to have an agonistic activity on the kappa-opioid receptor, and the uses as an analgesic and a diuretic have been disclosed (Patent document 6). Furthermore, the uses of the compound or the acid addition salt as an antitussive (Patent document 7), a brain cell protective agent (Patent document 8), an antipruritic (Patent document 9), a therapeutic agent for hyponatremia (Patent document 10), an ORL-1 receptor antagonist (Patent document 11), a therapeutic agent for neuropathic pain (Patent document 12), an antipruritic for cornea or conjunctiva (Patent document 13), a therapeutic agent for neuropsychiatric disorder (Patent document 14), a therapeutic agent for drug dependence (Patent document 15), a therapeutic agent for sepsis (Patent document 16), a therapeutic agent for multiple sclerosis-associated pruritus (Patent document 17), a therapeutic agent for schizophrenia (Patent document 18), a therapeutic agent to improve skin conditions (Patent document 19) and a therapeutic agent for dyskinesia (Patent document 20) have been disclosed, and, though published after the priority date of the present application, the uses of the compound or the acid addition salt as a therapeutic agent for fibromyalgia (Patent document 21) and a therapeutic agent for biliary tract disease (Patent document 22) have also been disclosed. However, the therapeutic or prophylactic effect thereof for cachexia has not been disclosed at all.

### prior Art Documents

### Patent Documents

Patent document 1: JP 2000-95685 A
Patent document 2: JP Hei 5-43466 A
Patent document 3: PCT WO98/051329
Patent document 4: PCT WO05/097261
Patent document 5: PCT WO02/066475
Patent document 6: PCT WO93/015081
Patent document 7: PCT WO95/001178
Patent document 8: PCT WO95/003307
Patent document 9: PCT WO98/023290
Patent document 10: PCT WO99/005146
Patent document 11: JP 2000-53572 A
Patent document 12: PCT WO01/014383
Patent document 13: JP 2001-163784 A
Patent document 14: PCT WO02/078744
Patent document 15: PCT WO99/011289
Patent document 16: PCT WO02/089845
Patent document 17: PCT WO06/095836
Patent document 18: PCT WO09/001764
Patent document 19: PCT WO09/044883
,Patent document 20: PCT WO08/133297
Patent document 21: JP 2011-074018 A
Patent document 22: PCT WO11/093441

### Non-patent Documents

Non-patent document 1: J. Parenteral and Enteral Nutrition, 12, 286-298 (1988)
Non-patent document 2: Am. J. Med., 85, 289-291 (1988)
Non-patent document 3: J. Natl. Cancer Inst., 89, 1763-1773 (1997)
Non-patent document 4: J. Clin. Oncol., 12, 213-225 (1994)

US 7 652 025 B2 and WO 02/089845 A1 both disclose the use of epoxy-morphinans or of nalfurafine HCl for treating sepsis. WO 2011/009020 A2 discloses the preparation of epoxy-morphinans for use in treating cancer and ocular neovascular disorders. WO 2011/009020 A2 discloses the preparation of morphinans such as nalfurafine, their activity as TLR9 inhibitors and their uses in treating cancer.

US 2002/165247 A1 discloses the use of morphinan derivatives for treating i.a. cachexia.

### Summary of the Invention

### Problems to be Solved by the Invention

An object of the present invention is to provide a compound for use in the treatment or prevention of cancer cachexia, which comprises as an effective ingredient a compound having a morphinan skeleton or a pharmacologically acceptable acid addition salt thereof and is capable of preventing or ameliorating the progression of symptoms of cancer cachexia.

### Means for Solving the Problems

The present inventors intensively studied to solve the above-described problems and discovered that a specific compound having a morphinan skeleton or a pharmacologically acceptable acid addition salt thereof has an excellent therapeutic or prophylactic effect on cachexia, thereby completing the present invention.

That is, the present invention relates to the [4].

[1] A compound for use in the treatment or prevention of cancer cachexia comprising as an effective ingredient a compound represented by the following general Formula **(I)** is disclosed herein : 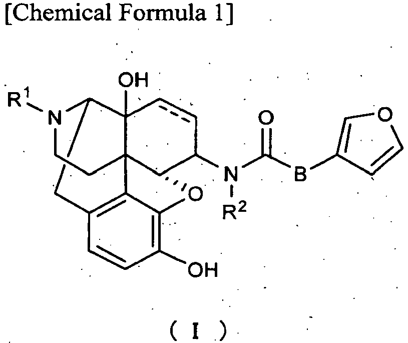 [wherein the double line consisting of a dashed line and a solid line represents a double bond or single bond, R¹ represents C₄-C₇ cycloalkylalkyl, R² represents C₁-C₅ linear or branched alkyl, and B represents -CH=CH-] or a pharmacologically acceptable acid addition salt thereof.
[2] The compound according to [1], wherein, in the general Formula (I), R¹ is cyclopropylmethyl, cyclobutylmethyl, cyclopentylmethyl or cyclohexylmethyl, and R² is methyl, ethyl or propyl is disclosed herein.
[3] The compound according to [1], wherein, in the general Formula (I), R¹ is cyclopropylmethyl, R2 is methyl, and B is -CH=CH- in trans-form. is disclosed herein.

[4] The compound according to [1], wherein the compound represented by the general Formula (I) is (-)-17-(cyclopropylmethyl)-3,14β-dihydroxy-4,5α-epoxy-6β-[N-methyl-trans-3-(3-f uryl)acrylamido]morphinan:

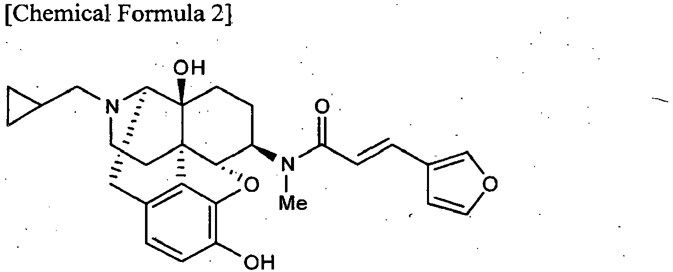

### Effects of the Invention

The present invention provides a compound for use in the treatment or prevention of cancer cachexia comprising as an effective ingredient the compound having a morphinan skeleton of chemical formula 2 or a pharmacologically acceptable acid addition salt thereof. The compound of the present invention ameliorates a systemic syndrome having predominant symptoms such as remarkedly body weight loss, anemia, edema, anorexia, general prostration, malaise and the like in chronic diseases.

### Brief Description of the Drawings

Fig. 1 shows a therapeutic effect of Compound 1 on cachexia (life-extending effect).
Fig. 2 shows a therapeutic effect of Compound 1 on cachexia (suppressive effect on body weight loss).

### Disclosure

The compound for use in the treatment or prevention of cachexia comprises as an effective ingredient a compound represented by the following general Formula (II) is disclosed herein: 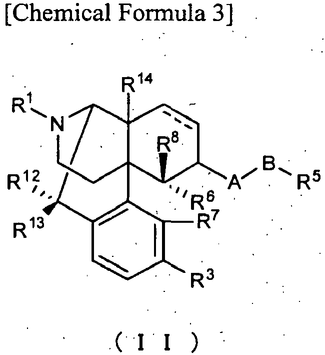
[wherein the double line consisting of a dashed line and a solid line represents a double bond or single bond;
R¹ represents C₁-C₅ alkyl, C₄-C₇ cycloalkylalkyl, C₅-C₇ cycloalkenylalkyl, C₆-C₁₂ aryl, C₇-C₁₃ aralkyl, C₄-C₇ alkenyl, allyl, furan-2-ylalkyl (wherein the alkyl moiety has 1 to 5 carbon atom(s)) or thiophen-2-ylalkyl (wherein the alkyl moiety has 1 to 5 carbon atom(s));
R¹⁴ represents hydrogen, hydroxy, nitro, C₁-C₅ alkanoyloxy, C₁-C₅ alkoxy, C₁-C₅ alkyl or NR⁹R¹⁰ (wherein R⁹ represents hydrogen or C₁-C₅ alkyl, and R¹⁰ represents hydrogen, C₁-C₅ alkyl or -C(=O)R¹¹ (wherein R¹¹ represents hydrogen, phenyl or C₁-C₅ alkyl));
R³ represents hydrogen, hydroxy, C₁-C₅ alkanoyloxy or C₁-C₅ alkoxy;
A represents XC(=Y)-, -XC(=Y)Z-, -X-or -XSO₂- (wherein X, Y and Z each independently represent NR⁴, S or O (wherein R⁴ represents hydrogen, C-₁-C₅ linear or branched alkyl, or C₆-C₁₂ aryl, and, in cases where two or more R⁴ exist in the formula, these may be the same or different));
B represents a valence bond, C₁-C₁₄ linear or branched alkylene (which may be substituted by at least one or more kinds of substituents selected from the group consisting of C₁-C₅ alkoxy, C₁-C₅ alkanoyloxy, hydroxy, fluorine, chlorine, bromine, iodine, amino, nitro, cyano, trifluoromethyl and phenoxy; and whose 1 to 3 methylene group(s) may be substituted by carbonyl), C₂-C₁₄ linear or branched acyclic unsaturated hydrocarbon containing 1 to 3 double bond(s) and/or triple bond(s) (which may be substituted by at least one or more kinds of substituents selected from the group consisting of C₁-C₅ alkoxy, C₁-C₅ alkanoyloxy, hydroxy, fluorine, chlorine, bromine, iodine, amino, nitro, cyano, trifluoromethyl and phenoxy; and whose 1 to 3 methylene group(s) may be substituted by, carbonyl), or C₁-C₁₄ linear or branched, saturated or unsaturated hydrocarbon containing 1 to 5 thioether bond(s), ether bond(s) and/or amino bond(s) (wherein no heteroatom is bound directly to A, and 1 to 3 methylene group(s) thereof may be substituted by carbonyl);
R⁵ represents hydrogen or an organic group having any of the following basic skeletons:

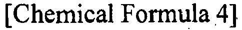

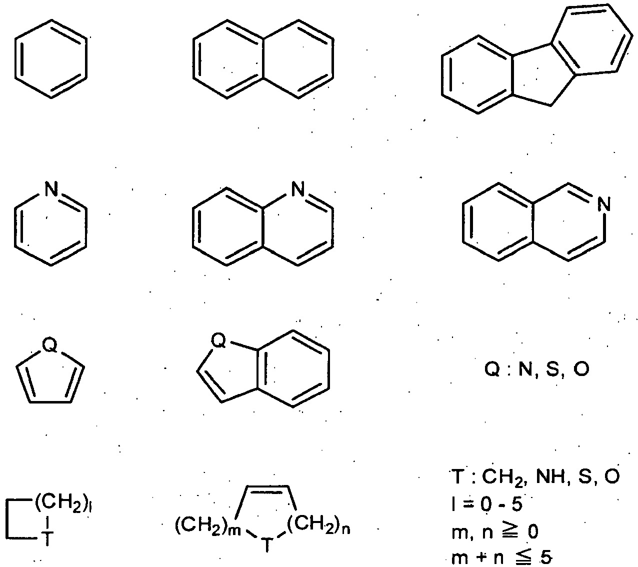

(wherein Q represents N, O or S; T represents CH₂, NH, S or O;1 represents an integer of 0 to 5; m and n each independently represent an integer of 0 to 5;
the sum of m and n is not more than 5; and each organic group may be substituted by at least one or more kinds of substituents selected from the group consisting of C₁-C₅ alkyl, C₁-C₅ alkoxy, C₁-C₅ alkanoyloxy, hydroxy, fluorine, chlorine, bromine, iodine, amino, nitro, cyano, isothiocyanate, trifluoromethyl, trifluoromethoxy and methylenedioxy);
R⁶ represents hydrogen, and R⁷ represents hydrogen, hydroxy, C₁-C₅ alkoxy or C₁-C₅ alkanoyloxy; or R⁶ and R⁷ together represent -O-, -CH2- or -S-;
R⁸ represents hydrogen, C₁-C₅ alkyl or C₁-C₅ alkanoyl;
R¹² and R¹³ represent both hydrogen; one of these represents hydrogen and the other represents hydroxy; or these together represent oxo;
the general Formula (II) includes (+), (-) and (±) isomers],
or a pharmacologically acceptable acid addition salt thereof.

Among the compounds represented by the general Formula (II) or pharmacologically acceptable acid addition salts thereof, a compound represented by the previously-described general Formula (I) or a pharmacologically acceptable acid addition salt thereof is preferably contained as an effective ingredient in the therapeutic or prophylactic agent of the present disclosure for cachexia.

The double line consisting of a dashed line and a solid line in the general Formula (I) represents a double bond or a single bond, and the double line is preferably a single bond.

R¹ in the general Formula (I) represents C₄-C₇ cycloalkylalkyl. Among those, cyclopropylmethyl, cyclobutylmethyl, cyclopentylmethyl and cyclohexylmethyl are preferred as R¹, and R¹ is especially preferably cyclopropylmethyl.

R² represents C₁-C₅ linear or branched alkyl. Among those, methyl, ethyl and propyl are preferred as R², and R¹ is especially preferably methyl.

B represents -CH=CH-. As B, -CH=CH- in trans-form is preferred.

As a compound represented by the general Formula (I), the compound in (-) isomer, wherein the double line consisting of a dashed line and a solid line is a single bond; R¹ is cyclopropylmethyl, R² is methyl, and B is -CH=CH- in trans-form, that is, (-)-17-(cyclopropylmethyl)-3,14β-dihydroxy-4,5α,-epoxy-6β-[N-methyl-trans-3-(3-furyl)acrylamido]morphinan: 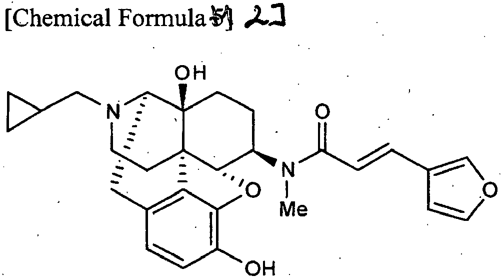 is especially preferred.

Compounds represented by the general Formula (I) or pharmacologically acceptable acid addition salts thereof can be produced in accordance with the method described in PCT WO93/015081.

Among those compounds represented by the general Formula (II), the compounds in which both R¹² and R¹³ represent hydrogen can be produced in accordance with the method described in PCT WO93/015081. Furthermore, the compounds in which R¹² and R¹³ together represent oxo can be produced in accordance with the methods described in Chem. Pharm. Bull., 52, 664-669 (2004) and PCT WO93/015081 from a compound, as a starting material, which can be produced in accordance with the methods described in, for example, Heterocycles, 63, 865-870 (2004) and Bioorg. Med. Chem. Lett., 5, 1505-1508 (1995). Moreover, the compounds in which R¹² represents hydroxy and R¹³ represents hydrogen can be produced in accordance with the method described in Chem. Pharm. Bull., 52, 664-669 (2004).

Examples of "pharmacologically acceptable acid addition salts" in the present invention include inorganic acid salts such as hydrochloric acid salt, sulfuric acid salt, nitric acid salt, hydrobromic acid salt, hydroiodic acid salt, phosphoric acid salt and the like; organic carboxylic acid salts such as acetic acid salt, lactic acid salt, citric acid salt, oxalic acid salt, glutaric acid salt, malic acid salt, tartaric acid salt, fumaric acid salt, mandelic acid salt, maleic acid salt, benzoic acid salt, phthalic acid salt and the like; organic sulfonic acid salts such as methanesulfonic acid salt, ethanesulfonic acid salt, benzenesulfonic acid salt, p-toluenesulfonic acid salt, camphorsulfonic acid salt and the like; and the like. Among these, hydrochloric acid salt, hydrobromic acid salt, phosphoric acid salt, tartaric acid salt, methanesulfonic acid salt and the like are preferably used.

"Cachexia" in the present invention includes a systemic syndrome having predominant symptoms such as remarkable body weight loss, anemia, edema, anorexia, general prostration, malaise and the like in chronic diseases such as malignant tumor, tuberculosis, diabetes, hematological disorders, endocrine disorders, infections, acquired immunodeficiency syndrome, etc. It includes, for example, cancer cachexia, tuberculous cachexia, diabetic cachexia, cachexia associated with a hematological disorder, cachexia associated with an endocrine disorder, cachexia associated with an infection, and cachexia associated with acquired immunodeficiency syndrome. The compound of the present invention is used for cancer cachexia associated with malignant tumor.

"Malignant tumor" (also referred to as cancer or malignant neoplasm) in the present invention includes "cancer (also referred to as carcinoma)" derived from epithelial tissue, "sarcoma" derived from non-epithelial tissue, and those derived from the hematopoietic organ. It includes, for example, malignant melanoma, malignant osteoma, stomach cancer, hepatoma, acute myeloid leukemia, acute lymphocytic leukemia, cervical cancer, uterus cancer, esophagus cancer, pancreatic cancer, prostate cancer, colon cancer, breast cancer, lung cancer, bladder cancer and ovarian cancer.

The compounds represented by the general Formula (I) disclosed herein or pharmacologically acceptable acid addition salts thereof have an ameliorating effect on cachexia, that is, an effect to ameliorate a systemic syndrome having predominant symptoms such as remarkable body weight loss, anemia, edema, anorexia, general prostration, malaise the like manifested in chronic diseases such as malignant tumor, tuberculosis, diabetes, hematological disorders, endocrine disorders, infections, acquired immunodeficiency syndrome, etc.

The compound of the present invention for cancer cachexia is used as a therapeutic or prophylactic agent for cachexia in mammals (for example, human, mouse, rat, rabbit, dog, cat, cattle, horse, swine, monkey and the like).

The compounds represented by the general Formula (I) or pharmacologically acceptable acid addition salts thereof are purified to quality levels suitable for medical application and, after passing a safety test, each of the compounds and acid addition salts thereof can be orally or parenterally administered as it is or as a pharmaceutical composition in admixture with a known pharmacologically acceptable acid(s), carrier(s), vehicle(s) and the like. A dosage form can be selected for oral administration from tablets, capsules, orodispersible tablets, powders, granules and the like; for parenteral administration from intravenous bolus injection, intravenous continuous infusion, intramuscular injection, subcutaneous injection, intradermal injection, tapes, patches and the like.

The content of the compounds represented by the general Formula (I) or pharmacologically acceptable acid addition salts thereof in a-pharmaceutical composition is not particularly limited, and the pharmaceutical composition can be formulated generally to contain 0.1 µg to 100 mg of any of the compounds and acid addition salts thereof per single dose. Furthermore, the dose of administration can be appropriately chosen depending upon the symptoms, age, sex and body weight of the patient, and its method of administration and the like, and the dose of the compounds represented by the general Formula (I) or pharmacologically acceptable acid addition salts thereof to be administered daily to an adult human is typically 0.1 µg to 20mg, preferably 1 µg to 10 mg, more preferably about 1 µg to 40 µg, which may be administered in a single dose or in separate doses.

As the compound disclosed herein for cancer cachexia, any of the compounds represented by the general Formula (I) or pharmacologically acceptable acid addition salts thereof may be administered either alone or in combination with one or more agents used for treatment or prevention of diseases, or for alleviation or reduction of symptoms. The agents to be combined may be low molecular weight compounds, or high molecular weight proteins, polypeptides, antibodies or vaccines, etc. In this case, the agent(s) in combination can be administered simultaneously with the agent of the present disclosure or acid addition salt thereof, or they can be administered sequentially with intervening time intervals. As a combination method, each agent may be used in combination or a drug combination may be formulated. The dose of the combined agents to be administered can be appropriately chosen based on the clinical dose of each agent. Furthermore, a combining ratio of the compound of the present disclosure for cancer cachexia and the agents to be combined can be appropriately chosen depending upon the subject of administration; the age, body weight and symptoms of the subject of administration; the period of administration, their dosage forms, their methods of administration, the combination of drugs, and the like.

The compound of the present invention can be used in combination with chemotherapeutic agents, immunotherapeutic agents, diuretic agents and the like.

Examples of the chemotherapeutic agents include alkylating agents such as cyclophosphamide, ifosfamide, melphalan, busulfan, nimustine, ranimustine, temozolomide and the like; antimetabolites of nucleic acid metabolism such as methotrexate, fluorouracil, tegafur, carmofur, doxifluridine, capecitabine, cytarabine, ancitabine, enocitabine, cytarabine ocfosfate, gemcitabine, mercaptopurine, fludarabine and the like; antitumor antibiotics such as doxorubicin, daunorubicin, pirarubicin, epirubicin, idarubicin, mitoxantrone, mitomycin C, bleomycin, peplomycin and the like; microtubule inhibitors such as vincristine, vinblastine, vindesine, vinorelbine, paclitaxel, docetaxel and the like; platinum based drugs such as cisplatin, carboplatin, nedaplatin and the like; topoisomerase inhibitors such as irinotecan, nogitecan, etoposide and the like; molecular targeted therapeutic agents such as trastuzumab, rituximab, imanitib and the like; or the like.

Examples of the immunotherapeutic agents include muramyl dipeptide and its derivatives, lentinan, sizofiran, ubenimex, picibanil, krestin, interferon, interleukin, granulocyte colony stimulating factor, erythropoietin and the like.

Examples of the diuretic agents include xanthine derivative drugs such as theobromine sodium salicylate; thiazide drugs such as ethiazide, cyclopenthiazide, trichlormethiazide, hydrochlorothiazide, hydroflumethiazide, benzyl hydrochlorothiazide, penflutizide, polythiazide, methyclothiazide; anti-aldosterone drugs such as spironolactone, triamterene and the like; carbonic anhydrase inhibitors such as acetazolamide and the like; chlorobenzene sulfonamide drugs such as chlortalidone, mefruside, indapamide and the like; azosemide, isosorbide, ethacrynic acid, piretanide, bumetanide, furosemide and the like.

The effectiveness of the compounds represented by the general Formula (I) or pharmacologically acceptable acid addition salts thereof in the treatment or prevention of cachexia, which are effective ingredients of the compound of the present invention for cachexia, can be evaluated by the effects thereof as described in Examples to prolong the lifetime or to suppress the body weight loss and the like in cancer-bearing animals.

### Examples

The present invention will be described hereinafter by way of Examples.

(Example 1) The life-extending effect and suppressive effect on body weight loss of (-)-17-(cyclopropylmethyl)-3,14β-dihydroxy-4,5α-epoxy-6β-[N-methyl-trans-3-(3-fu ryl)acrylamido]morphinan hydrochloride (Compound 1) in a cachexia model

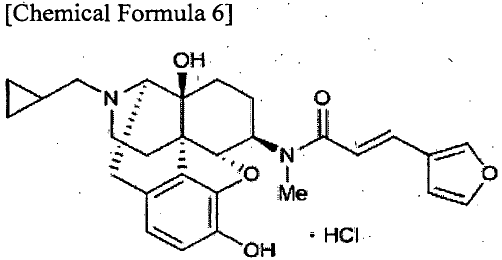

### (Compound 1)

The therapeutic effect of Compound 1 on cachexia was studied using an animal model of cachexia, the C57BL/6 strain mice transplanted with B16-F10 melanoma cells. Morphine and CI-977 were employed as comparison controls. Morphine is an analgesic widely used for the treatment of cancer pain and is a compound having a morphinan skeleton similar to that of the compounds of the present invention, while CI-977 as well as the compound of the present invention is a kappa opioid receptor agonist compound (Br. J. Pharmacol, 101, 183-189 (1990)).

C57BL/6 mice transplanted with B16-F10 melanoma cells develop the symptoms of cachexia such as body weight loss, reduced amount of movement the like as the tumor grows. The effect of each kind of compound on the symptoms of cachexia, which was caused by B16-F10 melanoma cells, was measured by survival rate and percent change in body weight as indicators.

### 1. Experimental methods

Subculture of B16-F10 melanoma cells was performed using RPMI1640 medium containing 10% FCS. For drug evaluation, 4-week-old male C57BL/6 mice (Japan SLC, Inc.) were purchased and used after 3-week acclimatization. Preparation of animal model of cachexia was performed as follows: 4x10⁵ B16-F10 melanoma cells per mouse were transplanted into a footpad of a mouse; it was verified 3 weeks after transplantation that the tumor volume had increased to some extent, and thus an animal model of cachexia was obtained. Also, the tumor volume was measured for each transplanted mouse and then the mice were divided into several groups such that the average tumor volume of each group would be equal. The evaluation of the therapeutic effects on cachexia was performed as described below.

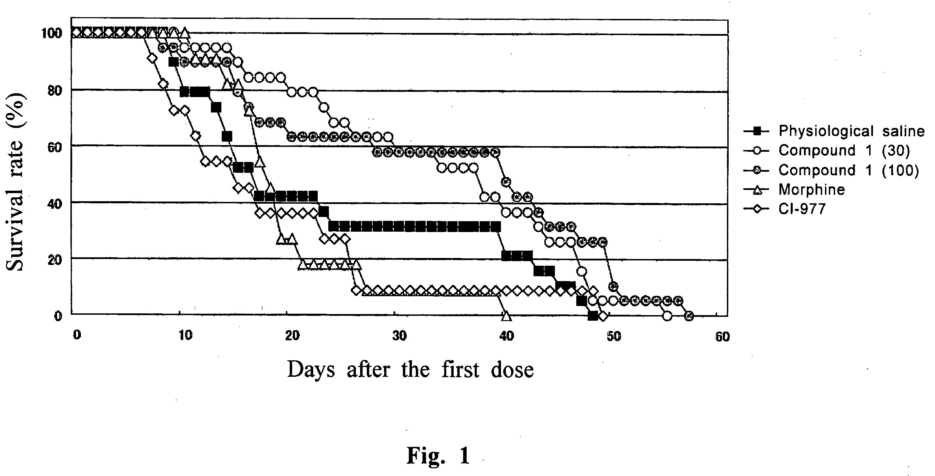

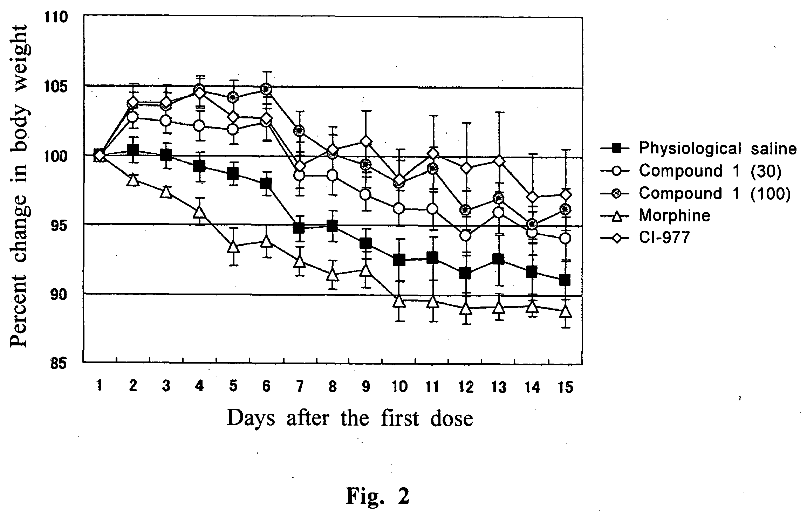

In the evaluation of each kind of compound for the life-prolonging effect and suppressive effect on body weight loss, each kind of compound was administered daily (the day of first administration was considered day 1; the compounds were not administered only on day 16) to the animal models after the grouping (3 weeks after the transplantation of the cells), and the lifetime and change in body weight of each animal model was observed. The dose of each kind of compound was as follows: 30 and 100 µg/kg for Compound 1 (n = 19, respectively; the numerical values in the parentheses shown in Figures indicate the doses), 5 mg/kg for morphine (n = 11), 100 µg/kg for CI-977 (n = 11). Physiological saline as a control was administered to the animal models (n = 19) in the same manner. The results observed until all the subjects died (day 57) were used to analyze the life-prolonging effect. Survival rate (%) indicates a ratio (%) of animals alive at each time point to all animals at the start of administration. Furthermore, the results observed until day 15 were used to analyze the suppressive effect on body weight loss. Percent change in body weight indicates a body weight at each time point expressed in percent, where the body weight on day 1 is defined as 100%. The results of the observation were shown in Figures 1 and 2.

### 2. Results

A statistically significant life-prolonging effect was demonstrated by administration of Compound 1 in comparison with the lifetime of the group administered with physiological saline (p = 0.0335, estimation of survival curves by Kaplan-Meier method and comparison by log-rank test). In contrast, the administration of CI-977 and morphine showed a tendency to redure the lifetime. A statistically significant suppressive effect on body weight loss was demonstrated by administration of Compound 1 and CI-977 in comparison with the body weight loss in the group administered with physiological saline (p = 0.0365 for 30 µg/kg of Compound 1, p = 0.0007 for 100 µg/kg of Compound 1, p = 0.0009 for CI-977; multiple comparisons over time (Dunnett's comparison)), while the administration of morphine showed a tendency of further body weight loss.

Thus, the compounds of the general Formula (I), as represented by Compound 1, or pharmacologically acceptable acid addition salts thereof demonstrated effects on both life prolongation and suppression of body weight loss, indicating the effectiveness as a therapeutic or prophylactic agent for cachexia.

## Claims

1. A compound for use in the treatment or prevention of cancer cachexia comprising as an effective ingredient a compound represented by (-)-17-(cyclopropylmethyl)-3,14β-dihydroxy-4,5α-epoxy-6β-[N-methyl-trans-3-(3-furyl)acrylamido]morphinan represented by Chemical Formula 2: 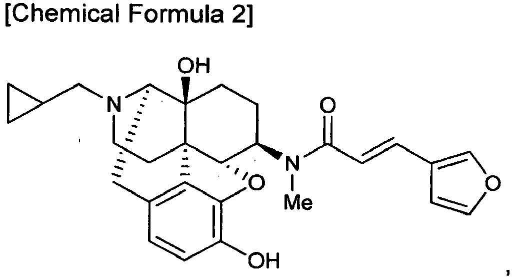 or a pharmacologically acceptable acid addition salt thereof.

## Patentansprüche

1. Verbindung zur Verwendung bei der Behandlung oder Vorbeugung einer Tumorkachexie, die als einen wirksamen Bestandteil eine Verbindung umfasst, die durch (-)-17-(Cyclopropylmethyl)-3,14β-dihydroxy-4,5α-epoxy-6β-[N-methyl-trans-3-(3-furyl)acrylamido]morphinan repräsentiert wird, das durch die chemische Formel 2 repräsentiert wird: 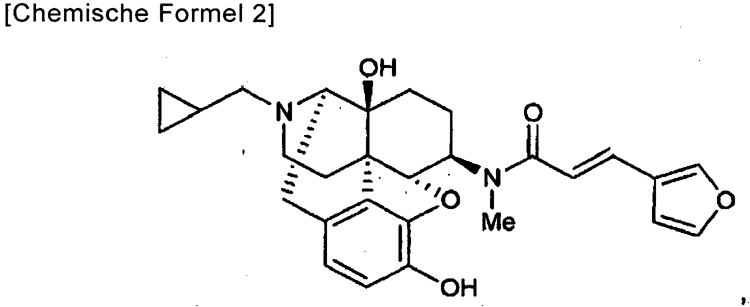 oder ein pharmakologisch akzeptables Säureadditionsalz davon.

## Revendications

1. Composé à utiliser dans le traitement ou la prévention de la cachexie due au cancer comprenant en tant que substance efficace un composé représenté par le (-)-17-(cyclopropylméthyl)-3,14β-dihydroxy-4,5α-époxy-6β-[N-méthyl-trans-3-(3-furyl)acrylamido]morphinane représenté par la Formule chimique 2 : 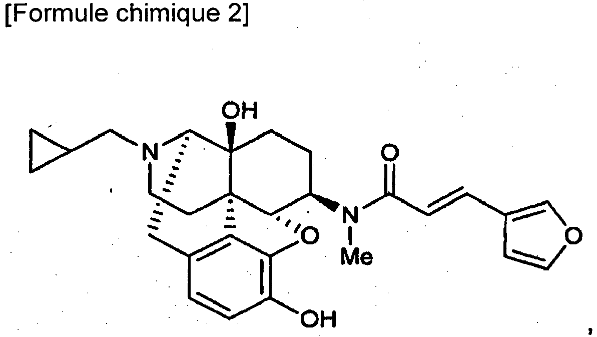 ou un sel d'addition acide pharmacologiquement acceptable de celui-ci.
